# EUROPEAN PATENT APPLICATION

(11) **EP 1 611 910 A1**
(43) Date of publication of application: **04.01.2006**
(21) Application number: 05105209.0
(22) Date of filing: 14.06.2005
(51) Int. Cl.: A61M 5/162, A61M 39/16, A61J 1/00

(54) **Infusion unit comprising a shell-type water-tightening casing**

(30) Priority: 21.06.2004 IT MI20041237
(71) Applicant: Bluehill Trust Reg, 9490 Vaduz (LI)
(72) Inventor: Gobbi Frattini, Paolo Giuseppe, 23035, Sondalo (IT)
(74) Representative: Mittler, Enrico

(57) **Abstract**

Unit for the infusion administration of liquid or dissolved active substances preserved in a storage container (1) is described, provided with a pierceable plug (2) of elastomeric material having predetermined thickness, comprising a sterilized bag (3), a tubular perforator (4) and a tube (5) for the connection between the bag (3) and the perforator (4). Said bag (3) contains a solution injectable by infusion to a patient and said tubular perforator (4) extends mainly along its longitudinal axis and it is provided with one tip (7) on one end to perforate said plug (2), which tip has holes in order to transfer said active substances from said storage container (1) to said bag (3) in order to form a final infusion solution. The unit comprises in addition a shell-type tightening casing (10) made up of two complementary halves closable on one another in order to enclose the zone of connection between the tube (5) and the container (1), including the perforator (4), and to guarantee the water-tightness of the unit in the aforesaid zone.

## Description

The present invention concerns a unit for the infusion administration of substances, as for example drugs or food integrators, comprising a shell-type water-tightening casing.

When active substances or solutions of active substances, as for instance drugs or food integrators, which in the continuation of this description will be indicated for simplicity by the term "active substances ", are administered by infusion, it is normal praxis to use sterilized bags previously filled with physiological solutions injectable by infusion to a patient. These bags have two openings on their bottom edge, in which two tubes get inserted; through the first one of the these tubes active substances get transferred from a storage container to the bag, where they get mixed with the injectable solution. If the active substances present in the storage container are, for example, powders, part of the liquid contained in the bag gets pressed by the bag into the container, where it gets used to melt the solid substances, afterwards the resulting solution is brought back into the bag, by taking advantage of the higher pressure created in the container.

It is through the second tube that the resulting solution, that in the continuation of this description will be indicated by the term "final solution", is administered to the patient.

In order to carry out the first stage, that is the transfer of the active substances from the storage container to the bag containing the injectable solution, the tube destined to this operation is normally provided, on its free end, with a tip having a plurality of holes, which perforates a plug of elastomeric material normally utilised to seal the containers of active substances and it emerges inside the latter in order to allow the injectable solution to be pressed, if necessary, into the container. The system then is simply turned upside down and the active substance gets transferred from the container to the bag, through the aforementioned tip and tube.

A unit of this kind is described in EP-A-0820777.

At the moment in which the unit gets turned upside down it is possible that, in the connection zone adjacent the plug of the container, there is a loss of active substance, which can get in contact with the patient or the nurse.

The active substance can be toxic, therefore it is opportune that the aforesaid unit does not have any loss.

Object of the present invention is to provide a unit for the infusion administration of substances of the above described type, that is capable to guarantee the absence of losses of active substance during the various stages of utilisation of the same unit.

According to the invention such scope is attained with a unit for the infusion administration of a liquid or of dissolved active substances preserved in a storing container, provided with an pierceable plug made of elastomeric material having predetermined thickness, comprising a sterilized bag, a tubular perforator and a connection tube between the bag and the perforator, in which said bag contains a solution injectable by infusion to a patient and said tubular perforator extends mainly along its longitudinal axis and it is provided with a tip to perforate said plug on one end, which tip has holes to transfer said active substances from said storage container to said bag in order to form a final infusion solution, the unit being characterised in that it further comprises a shell-type water-tightening casing made up of two closable halves complementary with each other in order to enclose the zone of connection between the tube and the container, including the perforator, and to guarantee the water-tightness of the unit in the aforesaid zone.

Said shell-type water-tightening casing allows therefore the manipulation of the unit at its various stages without any risk of loss of active substance and therefore without any danger for the patients and the nurses.

These and other characteristics of the present invention will become evident from the following detailed description an embodiment thereof which is illustrated as a non-limiting example in the enclosed drawings, in which:
Figure 1 shows a front view of the unit according to the present invention with the shell closed;
Figure 2 shows a partial front view of the unit with the shell open;
Figure 3 shows a view in section according to the line III-III in Figure 1.

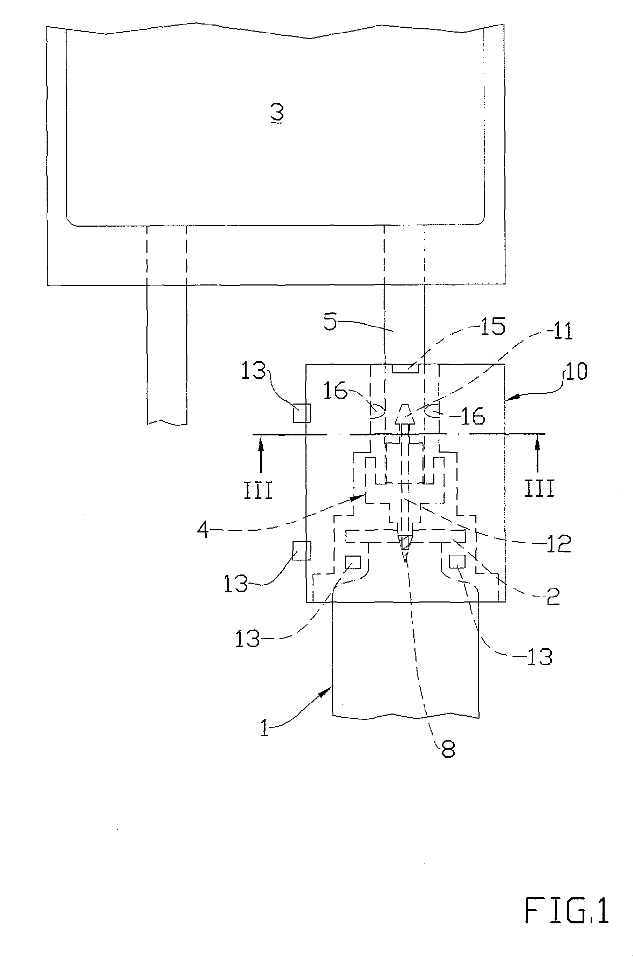

In Figure 1 a unit comprising a bag 3 for injectable physiological solution is shown, from which a tube 5 extends which through a tubular perforator 4 gets connected with a container of active substance 1 provided with a pierceable plug 2.

Said perforator 4 is internally passed through in longitudinal direction by a channel 12 between one first end provided with a pierced self-threading perforating tip 7, that is used to pierce the plug 2, and a second end which has a frangibile closing element 11.

In order to start the mixing of the substances present in the bag 3 and in the container 1, the unit gets arranged in such a way as to have the container 1 on the top and the bag 3 on the bottom, and the frangibile element 11 gets broken manually.

In order to prevent that there are losses during the flow of the active substance toward the bag 3, a water-tightening casing 10 is placed in the zone of connection between the tube 5 and the container 1.

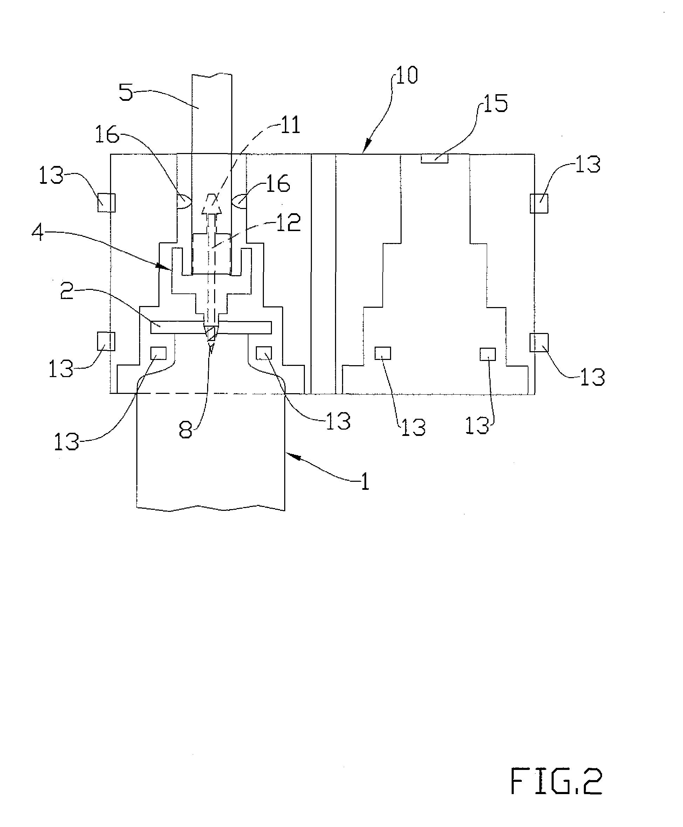

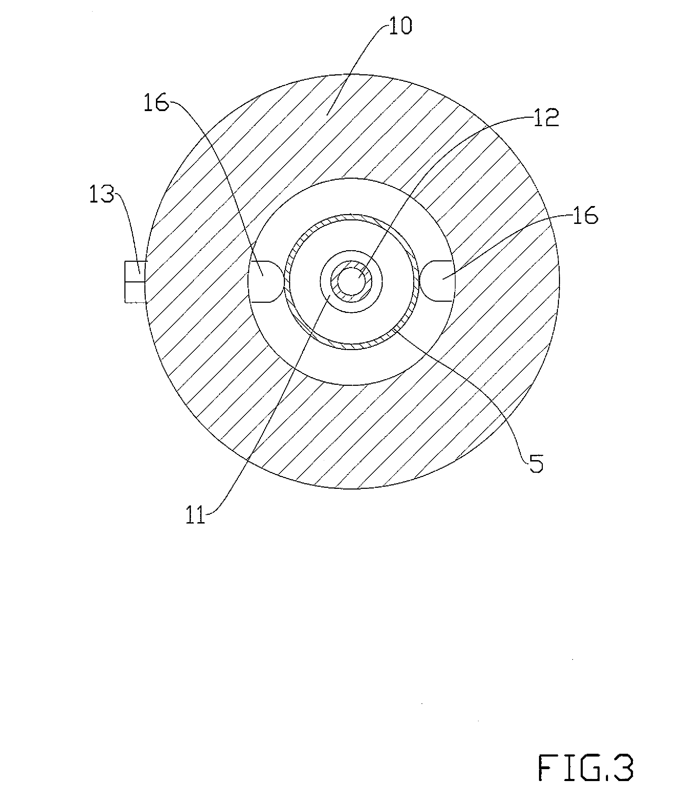

The water-tightening casing 10, which has the shape of a shell, is made up of two complementary halves 14 (Figure 2), which are closable one on the other one and around the aforesaid zone of connection between the tube 5 and the container 1 and lockable in closing position (Figures 1 and 3) by means of locking means 13, preferably of the permanent type. The two complementary halves 14 also provide centering means 16 and one of them includes in addition a projecting element 15 suitable to close the tube 5.

If the active substance is in the form of powder the liquid of the bag 3 is preliminarily made flow into the container 1, by turning the unit upside down and by squeezing the bag 3. In this way the mixing of the injectable solution with the active substance takes place.

The final solution is then sent back, by turning upside down the unit once again, into the bag 3, that is ready to be used by the patient.

## Claims

1. Unit for the infusion administration of a liquid or of dissolved active substances preserved in a storage container (1), provided with a pierceable plug (2) of elastomeric material having predetermined thickness, comprising a sterilized bag (3), a tubular perforator (4) and a tube (5) for the connection between the bag (3) and the perforator (4), in which said bag (3) contains a solution injectable by infusion to a patient and said tubular perforator (4) extends mainly along its longitudinal axis and it is provided with one tip (7) on one end to perforate said plug (2), which tip (7) has holes in order to transfer said active substances from said storage container (1) to said bag (3) so as to form a final infusion solution, the unit being **characterised in that** it further comprises a shell-type tightening casing (10) made up of two complementary halves closable one onto the other one in order to enclose the zone of connection between the tube (5) and the container (1), including the perforator (4), and to guarantee the water-tightness of the unit in the aforesaid zone.

2. Unit according to claim 1, **characterised in that** said tubular perforator (4) comprises in addition a frangibile closing element (11) which closes an axial channel (12), which longitudinally runs through the entire perforator (4), which, when the closing element gets removed, allows the mixing of the liquids and/or substances contained in the bag (3) and in the container (1).

3. Unit according to claim 1, **characterised in that** said shell-type casing (10) comprises means (13) for the locking of the two halves (14) in closing position.

4. Unit according to claim 1, **characterised in that** said shell (10) comprises in addition means (15) for the closing of the tube (5).
